# FASCICULE DE BREVET EUROPEEN

(11) **EP 3 386 999 B1**
(45) Date de publication et mention de la délivrance du brevet: **27.01.2021**
(21) Numéro de dépôt: 16825462.1
(22) Date de dépôt: 09.12.2016
(51) Int. Cl.: C07H 15/04, C07H 1/00, C11D 1/66, C11D 3/22, A61K 8/60, A61Q 19/10

(54) **PROCEDE DE PREPARATION DE COMPOSITIONS COMPRENANT DES ALKYL GULOSIDE URONATES D'ALKYLE, LESDITES COMPOSITIONS ET LEUR UTILISATION EN TANT QU'AGENT TENSIOACTIF**
VERFAHREN ZUR HERSTELLUNG VON ZUSAMMENSETZUNGEN (ALKYLGULOSID)ALKYL URONSÄUREDERIVATE ENTHALTEND, DIESE ZUSAMMENSETZUNGEN UND IHRE VERWENDUNG ALS TENSID
METHOD FOR THE PREPARATION OF A COMPOSITION COMPRISING DERIVATIVES OF ALKYL URONIC ACIDS OF ALKYL GULOSIDE, SAID COMPOSITIONS AND APPLICATIONS THEREOF AS SURFACTANT

(30) Priorité: 11.12.2015 FR 1562232
(43) Date de publication de la demande: 17.10.2018
(73) Titulaire: École Nationale Supérieure De Chimie, 35000 Rennes (FR)
(72) Inventeur: BENVEGNU, Thierry, 35000 Rennes (FR); PESSEL, Freddy, 56700 Merlevenez (FR); BENOÎT, Maud, 22620 Ploubazlanec (FR); LELONG, Yves, 22470 Plouezec (FR)
(74) Mandataire: Novagraaf Technologies
(86) Numéro de dépôt international: PCT/FR2016/053291
(87) Numéro de publication internationale: WO 2017/098175

(56) Documents cités:
- EP-A1- 0 635 516
- EP-A2- 0 537 820
- EP-A2- 0 819 698
- WO-A2-03/099870
- WO-A2-03/104248
- US-A- 5 147 861
- IKEDA A ET AL: "Preparation of low-molecular weight alginic acid by acid hydrolysis", CARBOHYDRATE POLYMERS, APPLIED SCIENCE PUBLISHERS, LTD. BARKING, GB, vol. 42, no. 4, 1 août 2000 (2000-08-01), pages 421-425, XP004195937, ISSN: 0144-8617, DOI: 10.1016/S0144-8617(99)00183-6
- INGOLF MACHER ET AL: "Methyl .alpha.- and .beta.-L-gulofuranosidurono-6,3-lactone", CARBOHYDRATE RESEARCH, PERGAMON, GB, vol. 77, no. 1, 1 janvier 1979 (1979-01-01), pages 225-230, XP002556806, ISSN: 0008-6215

## Description

### Domaine technique

La présente invention se rapporte à un nouveau procédé de préparation de compositions comprenant des alkyl guloside uronates d'alkyle ou un mélange d'alkyl guloside uronates d'alkyle et d'alkyl mannoside uronates d'alkyle, à partir exclusivement de matières premières biosourcées (alginates, oligoalginates, poly(oligo)guluronates, algues brunes) ou biocompatibles/biodégradables, et également de sels et acides de ceux-ci.

La présente invention trouve par exemple des applications dans les tensioactifs, notamment pour la cosmétique, le domaine phytosanitaire, la détergence et le BTP (Bâtiment et Travaux Publics).

Dans la description ci-dessous, les références entre crochets ([ ]) renvoient à la liste des références présentée à la fin du texte.

### État de la technique

Les tensioactifs 100% bio-sourcés composés d'une partie hydrophile et d'une partie lipophile toutes deux d'origine végétale représentent environ 5 à 10% des volumes totaux de tensioactifs. Malgré un fort potentiel dans les secteurs de la cosmétique, détergence en passant par les produits phytosanitaires, ils peinent à remplacer leurs équivalents d'origine fossile. Différence de prix et spectre d'applications plus étroit expliquent notamment cet écart. L'utilisation de matières premières à base de polysaccharides d'algues qui sont caractérisés par une fonctionnalité chimique originale par rapport aux polysaccharides issus de végétaux terrestres, constitue une approche qui pourrait permettre d'élargir les domaines d'applications des tensioactifs 100% bio-sourcés ainsi obtenus.

Les alginates, polysaccharides présents dans la paroi cellulaire des algues brunes sont constitués d'acides L-guluronique et D-mannuronique. Si des travaux ont déjà montré la possibilité de valoriser des oligomères de l'acide D-mannuronique dans le domaine des tensioactifs, la préparation de compositions à base de l'acide L-guluronique ou de mélanges à la fois d'acide L-guluronique et d'acide D-mannuronique sous forme monomère et permettant de valoriser la totalité des saccharides présents dans le biopolymère n'a pas été développée à ce jour.

Les tensioactifs glycosidiques constituent une famille importante de tensioactifs bio-sourcés commerciaux. Ce sont des tensioactifs dont les propriétés de surface, la biodégradabilité et l'innocuité vis-à-vis de la peau leur offrent de nombreuses applications dans les domaines de la détergence de la cosmétique et de l'alimentaire. Les tensioactifs non ioniques dérivés de sucres sont principalement représentés sur le marché par les alkylpolyglucosides APG (85 000t/an), les esters de sorbitan (20 000t/an), les sucroesters (<10 000t/an) et les méthylglucosides (<10 000t/an). Les tensioactifs anioniques dérivés de sucres occupent une place beaucoup moins importante comparativement à leurs homologues non ioniques (Sugar-based Surfactants : fundamentals and applications », Surfactant science series vol.143, Ed. C. Carnero Ruiz, CRC Press Taylor & Francis Group, 2009 (ISBN 978-1-4200-5166-7)) [1]. Ce constat peut notamment s'expliquer par la difficulté à mettre au point des procédés économiquement viables d'introduction contrôlée d'une ou plusieurs fonctions anioniques sur la structure saccharidique. En série carboxylate, les stratégies reposent principalement sur l'oxydation directe ou d'estérification du groupe C₆-OH de polyglycosides d'alkyle (APG). Un alkyl polyglucoside carboxylate Plantapon LGC Sorb (INCI-name sodium lauryl glucose carboxylate (and) lauryl glucoside) a été introduit sur le marché par Cognis en tant que nouveau tensioactif anionique pour des applications dans des formulations de soins corporels. Dans les shampoings et les gels douche, il apporte un meilleur pouvoir moussant que les tensioactifs non ioniques. Pour les soins du corps, il améliore les propriétés sensorielles. Un procédé industriel reposant sur la réaction du monochloroacétate de sodium dans une solution aqueuse d'alkyl polyglycoside (sans solvant additionnel) a été mis au point dans ce cadre (Behler et al., in Proceedings 6th World Surfactant Congress, CESIO June, Berlin, 2004) [2].

Actuellement, il existe peu de voies d'accès à des tensioactifs 100% bio-sourcés sous forme de monomère non-ionique et anionique à partir d'une même source naturelle. Des tensioactifs dérivés d'acides glycosiduroniques ont été produits en série glucu- et galacturonique sous forme de mono- et d'oligomères (Brevets EP 0532370 et US 5,312,907, Demande de Brevet FR 2717177, Demande internationale WO 9302092, Brevet EP 0819698) [3-6]. En série guluronique, les tensioactifs existent sous la forme polymérique, en particulier sous forme d'alginate modifié (Demande internationale WO 9812228, Brevet US 5,147,861) [7, 8]. Des tensioactifs dérivés d'acide (alkyl-D-mannopyranoside) uronique ont été produits sous forme mono- et dimérique à partir d'oligomères de l'acide D-mannuronique (Benvegnu et al., Topics in Current Chemistry, 294 : 143-164, 2010; Roussel et al., Eur. J. Org. Chem., 3085-3094, 2005 ; Demande de Brevet FR 02/840306 ; Demande internationale WO 03/104248) [9-12]. Le procédé repose sur la production d'oligomannuronates saturés (dépolymérisation acide) et insaturés (dépolymérisation enzymatique) qui sont ensuite transformés en intermédiaires monosaccharide et disaccharide comportant 2 (monosaccharide) ou 4 (disaccharide) chaînes butyle. Ces synthons constituent des intermédiaires clefs à la synthèse éco-compatible (sans solvant, sans rejet, réactifs biodégradables) de structures tensioactives de balance hydrophile-hydrophobe variable, obtenues par un procédé de transestérification/transglycosylation par des alcools gras de longueur variable. Ces tensioactifs possédant des chaînes lipophiles identiques (deux chaînes dans le cas du monosaccharide) peuvent être ensuite saponifiés pour accéder à des tensioactifs anioniques (une seule chaîne dans le cas du monosaccharide) comportant un motif carboxylate. Ces molécules amphiphiles couvrent plusieurs applications dans le domaine de la détergence et des cristaux liquides.

La Demande internationale WO 03/099870 [13] déposée par le CEVA porte sur la préparation d'oligo mannuronique et oligo guluronique à partir d'algues fraîches ou sèches. Ce procédé consiste en une extraction préalable des alginates suivi de nombreuses étapes de précipitation par modulation du pH du milieu réactionnel afin de séparer les blocs G et les blocs M constitutifs des alginates. Enfin une étape d'hydrolyse enzymatique ou acide conduit aux oligo mannuroniques ou oligo guluroniques.

Il existe donc un réel besoin d'un procédé palliant ces défauts, inconvénients et obstacles de l'art antérieur, en particulier d'un procédé simple permettant de synthétiser des compositions tensioactives directement à partir de matières premières moins raffinées, de réduire les coûts et d'améliorer les propriétés attendues dans le domaine des tensioactifs.

### Description de l'invention

Les inventeurs ont mis au point un nouveau procédé, sans solvant, utilisant des réactifs biocompatibles / biodégradables, pour accéder directement et simplement à des compositions non ioniques et anioniques de dérivés de l'acide L-guluronique ou de mélanges d'acides L-guluronique et de D-mannuronique (forme monomère) directement à partir d'oligoalginates, d'alginates raffinés et semi-raffinés (mélange d'alginate, de cellulose, d'hémicellulose, de laminarane et de fucane). Les poly(oligo)guluronates (ou blocs homopolymériques d'acide α-L-guluronique, en partie sous forme de sel de sodium) proviennent de la dépolymérisation d'alginates selon le procédé décrit dans la Demande internationale WO 03/099870 [13]. Les alginates et les oligoalginates sont obtenus par des traitements simples en milieux aqueux acide, à partir d'algues fraiches ou sèches, par exemple obtenus selon le protocole décrit dans l'exemple 2 ci-après selon le procédé de la Demande internationale WO 98/40511 [15]. Des traitements simples de purification partielle des bruts réactionnels (élimination des sels et/ou les alcools gras résiduels) ou d'isolement des compositions tensioactives conduisent aux composés dérivés et aux compositions présentant des performances/caractéristiques en adéquation avec les besoins du marché.

La présente invention a donc pour objet un procédé de préparation d'une composition comprenant :
(i) des alkyl guloside uronates d'alkyle de formules (Ia), (Ib) et (Ic) : 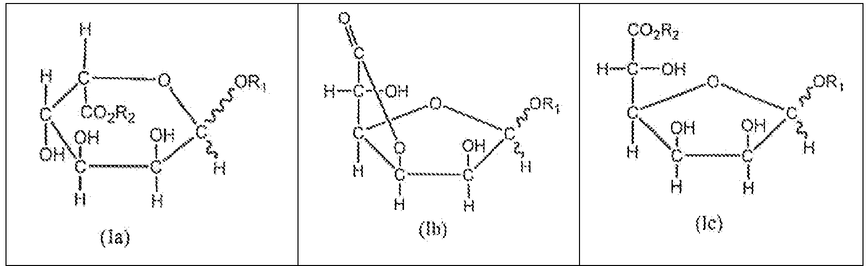 ou
(ii) un mélange d'alkyl guloside uronates d'alkyle de formule (Ia, Ib et Ic) et d'alkyl mannoside uronates d'alkyle de formule (IIa), (IIb) et (IIc) : 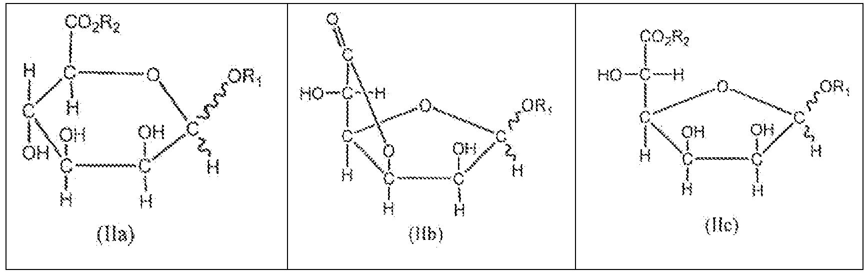 où
   - R₁ est une chaîne alkyle de 2 à 22 atomes de carbone, linéaire ou ramifiée, saturée ou insaturée ;
   - R₂ est un atome d'hydrogène, R₁, un atome de métal alcalin, un atome de métal alcalino-terreux, ou un groupe ammonium quaternaire de formule (III) : 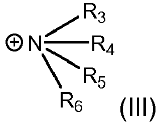
   - où chacun des R₃ à R₆ est indépendamment un atome d'hydrogène, un alkyle ayant de 1 à 6 atomes de carbone ou un hydroxyalkyle ayant de 1 à 6 atomes de carbone, et caractérisé en ce que ledit procédé comprend :
      a) une étape d'hydrolyse de poly(oligo)guluronates, d'oligoalginates et/ou d'alginates ;
      b) une étape d'estérification et glycosylation de l'hydrolysat issu de l'étape a) par un alcool de formule ROH, linéaire ou ramifié, saturé ou insaturé, ayant de 1 à 4 atomes de carbone ;
      c) une étape de trans-estérification et trans-glycosylation du milieu réactionnel issu de l'étape b) par un alcool de formule R'OH, linéaire ou ramifié, saturé ou insaturé, ayant de 2 à 22 atomes de carbone ;
      d) une étape de neutralisation du milieu réactionnel issu de l'étape c) en présence d'eau et d'une base M(OH)x dans laquelle M est un métal alcalin ou alcalino-terreux, et x est la valence.

Les compositions non ioniques et anioniques contiennent de façon inédite sous la forme de monomère des dérivés de l'acide L-guluronique ou des deux acides uroniques (acide L-guluronique et acide D-mannuronique) issus du même polysaccharide et dans certains cas des hexoses et pentoses dérivés d'autres polysaccharides présents dans l'extrait d'algues (cellulose, hémicellulose, laminarane, fucane : dans le cas d'alginates semi-raffinés comme matière première).

Par « poly(oligo)guluronates » au sens de la présente invention, on entend des blocs homopolymériques d'acide α-L-guluronique en partie sous forme de sel de sodium issus de la dépolymérisation d'alginates, par exemple selon le procédé de la Demande Internationale WO 03/099870 [13].

Par « oligoalginates » au sens de la présente invention, on entend les produits issus d'un traitement par voie enzymatique et/ou acide d'alginate, par exemple obtenus selon le protocole décrit dans l'exemple 2 ci-après selon le procédé de la Demande internationales WO 98/40511 [15].

Par « alginates » au sens de la présente invention, on entend des alginates raffinés et/ou semi-raffinés, par exemple obtenus selon le protocole décrit dans l'exemple 2 ci-après. On entend également des alginates bactériens, obtenus par exemple à partir de culture de bactéries mucoïdes (*e.g*. cf. Demande internationale WO 2009/134368) [14].

Par « algues brunes » au sens de la présente invention, on entend les algues nommées *Phaeophyceae* ou Phéophycées dont il existe 1500 espèces (*e.g. Ascophyllum nodosum, Fucus serratus, Laminaria hyperborea, Laminaria digitata, Ecklonia maxima, Macrocystis pyrifera, Sargassum vulgare, etc...),* et dont les parois sont essentiellement composées de fucanes sulfatés et d'alginate.

Par « solvant ionique » au sens de la présente invention, on entend par exemple le chlorure de 1-butyl-3-méthylimidazolium [BMIM]Cl, le bromure de 1-butyl-3-méthylimidazolium [BMIM]Br, le méthylsulfate de tris-(2-hydroxyéthyl)méthylammonium (HEMA) et l'acétate de 1-éthyl-3-méthylimidazolium [EMIM]AcO ; ledit solvant ionique comprenant typiquement jusqu'à 10% d'eau.

Par « solvant eutectique » au sens de la présente invention, on entend des systèmes formés d'un mélange eutectique de bases ou d'acides de Lewis ou Brönsted qui peuvent contenir une variété d'espèces anioniques et/ou d'espèces cationiques. Les solvants eutectiques de première génération ont été basés sur des mélanges de sels d'ammonium quaternaire avec des donneurs de liaison d'hydrogène tels que les amines et les acides carboxyliques (*e.g.* sel d'ammonium quaternaire et (hydrate de) chlorure de métal.

Selon un mode de réalisation particulier de la présente invention, ledit procédé comprend, avant l'étape a), les étapes de préparation des alginates (semi-)raffinés, oligoalginates et poly(oligo)guluronates. Les poly(oligo)guluronates proviennent de la dépolymérisation d'alginates. Les alginates semi-raffinés proviennent de la lixiviation acide d'algues brunes suivie d'une solubilisation des alginates de sodium par augmentation du pH puis d'une séparation solide/liquide afin d'éliminer les résidus algaux. Les alginates raffinés proviennent d'une étape supplémentaire de dépigmentation par le formol et d'une étape de purification. Les oligoalginates proviennent du traitement par voie enzymatique et/ou acide de solution d'alginate.

Selon un mode de réalisation particulier de la présente invention :
(i) dans le cas des alginates (« mode séparé »), l'étape d'estérification et glycosylation b) du milieu réactionnel issu de l'étape a) est réalisée après l'étape a) du fait d'une solubilité insuffisante des polysaccharides dans un milieu aqueux contenant un alcool à chaîne courte (1 à 4 atomes de carbone).
(ii) dans le cas des poly(oligo)guluronates ou des oligoalginates, les étapes a) et b) sont réalisées en même temps (« mode combiné ») en raison de la solubilité suffisante de ces matières premières dans un milieu constitué essentiellement d'eau et d'un alcool à chaîne courte (1 à 4 atomes de carbone).

Selon un mode de réalisation particulier de la présente invention, pour les alginates, l'étape a) est réalisée en présence (i) d'eau et/ou d'un solvant ionique et/ou d'un solvant eutectique, et (ii) d'un catalyseur acide tel que par exemple l'acide chlorhydrique, l'acide sulfurique, un acide alkyl sulfurique tel que l'acide décyl ou lauryl sulfurique, un acide sulfonique tel que l'acide benzènesulfonique, l'acide paratoluène sulfonique, l'acide camphosulfonique, un acide alkylsulfonique tel que l'acide méthanesulfonique (ou méthylsulfonique), l'acide décylsulfonique, l'acide laurylsulfonique, l'acide sulfosuccinique ou un sulfosuccinate d'alkyl tel le sulfosuccinate de décyle ou sulfosuccinate de lauryle, les acides perhalohydriques, tel que l'acide perchlorique, des métaux tels que le fer, leurs oxydes ou leurs sels, comme leurs halogénures. De préférence, il s'agit d'acide alkylsulfonique ou d'acide méthanesulfonique.

L'étape a) en «mode séparé » est réalisée de façon distincte de l'étape b), par exemple, en mettant en présence 1 équivalent d'alginates raffinés ou semi-raffinés obtenus à partir des espèces *Ascophyllum, Durvillaea, Ecklonia, Laminaria, Lessonia, Macrocystis, Sargassum* et *Turbinaria,* et de préférence d'alginates obtenus à partir des espèces *Laminaria,* préférentiellement à partir de l'espèce *Laminaria digitata* dont la composition massique est pour l'alginate raffiné : matière sèche 94,2 %/brut, matière minérale 37,2 %sec/sec, acides uroniques (D-mannuronique et L-guluronique) 67,5 %sec/sec, et pour l'alginate semi-raffiné : matière sèche 94,9 %/brut, matière minérale 47,0 %sec/sec, acides uroniques (D-mannuronique et L-guluronique) 29,1 %sec/sec, glucose 10,9 %sec/sec, fucose 2,1 %sec/sec, xylose < 0,5 %sec/sec ; de 10 à 1000 équivalents en masse d'eau, de préférence 30 équivalents en masse ; de 10⁻³ à 10 équivalents molaires d'un catalyseur acide tel que défini ci-dessus, et de préférence de 1,1 à 10 équivalents molaires, d'acide alkylsulfonique, et de préférence 5 équivalents molaires d'acide méthanesulfonique. Cette étape d'hydrolyse des alginates (semi-)raffinés est préférentiellement chauffée entre 80 °C et 130 °C, et de préférence portée au reflux de l'eau à la pression atmosphérique sur un temps de réaction pouvant être de 1 à 24 heures et de préférence sur un temps de réaction de 8 heures.

Puis l'étape d'estérification et glycosylation b) du milieu réactionnel issu de l'étape a) en «mode séparé » est conduite à la pression atmosphérique et à la température d'ébullition de l'eau ou de l'azéotrope formé avec un alcool ROH, linéaire ou ramifié, saturé ou insaturé, ayant de 1 à 4 atomes de carbone. De préférence, l'alcool ROH est le n-butanol.

Cette étape d'estérification et glycosylation b) est réalisée, par exemple, en introduisant dans le milieu réactionnel issu de l'étape a) réalisée en présence d'alginates (semi-)raffinés de 2 à 300 équivalents molaires d'alcool et de préférence 150 équivalents molaires. La réaction est alors conduite au reflux de l'azéotrope à pression atmosphérique (montage Dean Stark), entre 130 et 140°C dans le cas du butanol, de préférence sur 7 ou 15 heures, le temps que la quasi-totalité de l'eau soit éliminée. Puis le milieu réactionnel brut est refroidi à température ambiante.

Selon un mode de réalisation particulier de la présente invention, pour les poly(oligo)guluronates et les oligoalginates, les étapes a) et b) sont réalisées en même temps, en présence (i) d'eau et/ou d'un solvant ionique et/ou d'un solvant eutectique, (ii) d'un alcool à chaine courte (1 à 4 atomes de carbone) tel que par exemple le n-butanol, et (iii) d'un catalyseur acide tel que par exemple l'acide chlorhydrique, l'acide sulfurique, un acide alkyl sulfurique tel que l'acide décyl ou lauryl sulfurique, un acide sulfonique tel que l'acide benzènesulfonique, l'acide paratoluène sulfonique, l'acide camphosulfonique, un acide alkylsulfonique tel que l'acide méthanesulfonique (ou méthylsulfonique), l'acide décylsulfonique, l'acide laurylsulfonique, l'acide sulfosuccinique ou un sulfosuccinate d'alkyl tel le sulfosuccinate de décyle ou sulfosuccinate de lauryle, les acides perhalohydriques, tel que l'acide perchlorique, des métaux tels que le fer, leurs oxydes ou leurs sels, comme leurs halogénures. De préférence, il s'agit d'acide alkylsulfonique ou d'acide méthanesulfonique.

Les étapes a) et b) en «mode combiné » sont réalisées, par exemple, en mettant en présence 1 équivalent de poly(oligo)guluronates ou d'oligoalginates obtenus à partir des espèces telles que *Ascophyllum, Durvillaea, Ecklonia, Laminaria, Lessonia, Macrocystis, Sargassum* et *Turbinaria* ; de 0,1 à 100 équivalents en masse d'eau, de préférence 2 équivalents en masse ; de 2 à 300 équivalents molaires d'alcool tel que le n-butanol, de préférence de 150 équivalents molaires; de 10⁻³ à 10 équivalents molaires d'un catalyseur acide tel que défini ci-dessus, et de préférence de 1,1 à 10 équivalents molaires, d'acide alkylsulfonique, et de préférence 2,2 équivalents molaires d'acide méthanesulfonique. La réaction est alors conduite au reflux de l'azéotrope à pression atmosphérique (montage Dean Stark), entre 130 et 140°C dans le cas du butanol, de préférence sur 7 ou 15 heures, le temps que la quasi-totalité de l'eau soit éliminée. Puis le milieu réactionnel brut est refroidi à température ambiante.

La composition ainsi formée à l'issue des étapes a) et b) réalisées en mode séparé ou combiné est constituée majoritairement de composés à deux chaînes provenant de l'alcool (de préférence du butanol) dérivés de l'acide L-guluronique (à partir de poly(oligo)guluronates) ou dérivés du mélange d'acide L-guluronique et d'acide D-mannuronique (à partir d'oligoalginates ou d'alginates (semi-)raffinés).

Selon un mode de réalisation particulier de la présente invention, la préparation d'une composition comprenant des dérivés de l'acide L-guluronique ou un mélange de dérivés d'acide L-guluronique et d'acide D-mannuronique, où la chaîne alkyle est plus longue, se poursuit par une étape de trans-estérification et trans-glycosylation c) réalisée sur cette composition constituée majoritairement du composé bicaténaire et issue de l'étape b), en présence d'un alcool de formule R'OH, linéaire ou ramifié, saturé ou insaturé, où R' est composé de 2 à 22, de préférence de 8 à 18, préférentiellement de 12 à 18, atomes de carbone. Par exemple, l'alcool R'OH est choisi dans le groupe constitué des alcools gras linéaires, saturés ou insaturés tels que le dodécanol et l'alcool oléïque. Cette étape de trans-estérification et trans-glycosylation c) est réalisée, par exemple, en introduisant dans le milieu réactionnel issu de l'étape b) (avec comme matière première les poly(oligo)guluronates, les oligoalginates) de 2 à 50 équivalents molaires d'un alcool de formule R'OH tel que défini ci-dessus, et de préférence de 4 équivalents molaires; de 10⁻³ à 10 équivalents molaires d'un catalyseur acide tel que défini ci-dessus, et de préférence de 0,1 à 10 équivalents molaires d'acide alkylsulfonique, et de préférence 1 équivalent molaire d'acide méthanesulfonique. Cette étape de trans-estérification et trans-glycosylation c) est réalisée, par exemple, en introduisant dans la composition issue de l'étape b) (avec comme matière première les alginates (semi-)raffinés), de 2 à 50 équivalents molaires d'un alcool de formule R'OH, tel que défini ci-dessus, et de préférence de 4 équivalents molaires. Les réactions de trans-estérification et trans-glycosylation sont ensuite poursuivies en permettant de recycler l'alcool à chaine courte ROH préalablement utilisé pour la formation de la composition riche en dérivés de l'acide L-guluronique ou en un mélange de dérivés de l'acide L-guluronique et de l'acide D-mannuronique. La réaction est conduite de 1 heure à 24 heures à une température de préférence de 70 °C et sous pression réduite pour le recyclage de l'alcool précédemment cité. La composition ainsi formée constitue un produit d'usage dérivé de l'acide L-guluronique et/ou de l'acide D-mannuronique tel qu'un agent hydrotrope, un détergent non-ionique ou un agent émulsifiant.

Les étapes suivantes diffèrent ensuite selon que l'on souhaite accéder à des compositions (1) d'alkyl mannoside uronates d'alkyle et/ou d'alkyl guloside uronates d'alkyle, ou (2) de sels d'acides alkyl mannoside uroniques et/ou d'acides alkyl guloside uroniques.

Selon un mode de réalisation particulier de la présente invention, une étape de neutralisation d) du milieu réactionnel issu de l'étape c), une fois ramené à température ambiante et à pression atmosphérique, peut être réalisée en présence (i) d'eau, et (ii) d'une base M(OH)x dans laquelle M est un métal alcalin ou alcalino-terreux, et x est la valence. Cette étape d) est réalisée, par exemple, en introduisant dans le milieu réactionnel issu de l'étape c) (avec comme matière première les poly(oligo)guluronates, les oligoalginates), une fois ramené à température ambiante et à pression atmosphérique, de 0 à 19 équivalents molaires d'une solution aqueuse contenant une base de formule M(OH)ₓ telle que définie ci-dessus, et de préférence 2,2 équivalents d'une solution d'hydroxyde de sodium 1N ; de 100 à 1000 équivalents molaires d'eau et de préférence 780 équivalents molaires. Cette étape d) est réalisée, par exemple, en introduisant dans le milieu réactionnel issu de l'étape c) (avec comme matière première les alginates (semi-)raffinés), une fois ramené à température ambiante et à pression atmosphérique, de 0 à 19 équivalents molaires d'une solution aqueuse contenant une base de formule M(OH)ₓ telle que définie ci-dessus, et de préférence 1,65 équivalents d'une solution d'hydroxyde de sodium 1N ; de 100 à 1000 équivalents molaires d'eau et de préférence 775 équivalents molaires. Ensuite, l'ensemble est chauffé à 80°C sous vive agitation pendant 15 min. Une fois le mélange revenu à température ambiante, la phase aqueuse est séparée de la phase organique. Cette dernière est enfin séchée par distillation azéotropique de l'eau à l'aide de butanol. L'excès d'alcool de formule R'OH présent dans le brut organique peut être éliminé partiellement ou totalement par distillation moléculaire. Après une éventuelle purification par chromatographie sur gel de silice (CH₂Cl₂/MeOH 97:3 puis 96:4 puis 90:10), un mélange de produits est obtenu. A titre d'exemple, dans le cas d'utilisation d'oligoalginate, la composition molaire est d'environ : n-alkyl α-D-mannopyranosiduronate d'alkyle 30% ; n-alkyl α-D-mannofuranosiduronate d'alkyle 5% ; n-alkyl α,β-D-mannofuranosidurono-6,3-lactone 10% ; n-alkyl α,β-L-gulopyranosiduronate d'alkyle 25% ; n-alkyl β-L-guiofuranosiduronate d'alkyle 10%; n-alkyl α,β-L-gulofuranosidurono-6,3-lactone 20%. A titre d'exemple, dans le cas d'utilisation d'alginate semi-raffiné issu de *Laminaria digitata,* la composition massique est d'environ : dérivés n-alkyl D-mannuronate et L-guluronate 60%, n-alkyl L-fucose 15%, n-alkyl α,β-D-glucopyranose 25%.

La présente invention a également pour objet un procédé de préparation d'une composition comprenant (i) des sels d'acides alkyl guloside uroniques ou (ii) un mélange de sels d'acides alkyl guloside uroniques et de sels d'acides alkyl mannoside uroniques, ledit procédé comprenant une réaction de saponification e) de l'ester issu de l'étape c) en présence (i) d'une base M(OH)x dans laquelle M est un métal alcalin ou alcalino-terreux, et x est la valence, ou (ii) d'une base 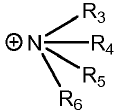 où chacun des R₃ à R₆ est indépendamment un atome d'hydrogène, un alkyle ayant de 1 à 6 atomes de carbone ou un hydroxyalkyle ayant de 1 à 6 atomes de carbone. De préférence, la base est choisie dans le groupe constitué de : l'hydroxyde de sodium, l'hydroxyde de potassium, l'ammoniaque ou un hydroxyde d'alkyl(hydroxyalkyl)ammonium. Cette étape de saponification e) de l'ester est réalisée, par exemple, en introduisant dans le milieu réactionnel issu de l'étape c) (avec comme matière première les poly(oligo)guluronates, les oligoalginates ou les alginates (semi-)raffinés), de 0,5 à 10 équivalents, et de préférence 1 à 3 équivalents, d'une base de formule M (OH)ₓ tel que définie ci-dessus. De préférence, la base M(OH)x est l'hydroxyde de sodium, l'hydroxyde de potassium, l'ammoniaque ou un hydroxyde d'alkyl (hydroxyalkyl) ammonium. La réaction de saponification des esters est préférentiellement conduite à des températures comprises entre 0 °C et 100 °C, et de préférence à 70 °C et pendant une durée de 15 min à plusieurs heures, et de préférence pendant une heure. A l'issue de la réaction, l'eau est ensuite éliminée par lyophilisation ou par distillation azéotropique avec du butanol. L'excès d'alcool de formule R'OH peut être éliminé partiellement ou totalement par distillation moléculaire ou par extraction solide-liquide avec un solvant organique, de préférence par extraction solide-liquide avec de l'acétone. Après une éventuelle purification par chromatographie sur gel de silice, un mélange de produits est obtenu. A titre d'exemple, dans le cas d'utilisation d'oligoalginate, la composition molaire est d'environ : *n*-alkyl α-D-mannopyranosiduronate de sodium 30% ; *n*-alkyl α,β-D-mannofuranosiduronate de sodium 15 % ; *n*-alkyl α,β-L-gulopyranosiduronate de sodium 25% ; *n*-alkyl α,β-L-gulofuranosiduronate de sodium 30%. A titre d'exemple, dans le cas d'utilisation d'alginate semi-raffiné issu de *Laminaria digitata,* la composition massique est d'environ : dérivés *n*-alkyl D-mannuronate de sodium et *n*-alkyl L-guluronate de sodium 45%, *n*-alkyl L-fucose 20%, *n*-alkyl α,β-D-glucopyranose 35%.

La présente invention a également pour objet un procédé de préparation d'une composition comprenant (i) des acides alkyl guloside uroniques ou (ii) un mélange d'acides alkyl guloside uroniques et d'acides alkyl mannoside uroniques, ledit procédé comprenant une réaction d'acidification f) des sels issus de l'étape e) en présence d'un acide choisi dans le groupe constitué de : l'acide chlorhydrique, l'acide sulfurique, l'acide oxalique, un acide sulfonique ou une résine sulfonique sous sa forme H⁺. Les sels inorganiques peuvent être éliminés par extraction liquide-liquide. Cette réaction d'acidification est réalisée, par exemple, en mettant en présence 1 équivalent de sels issus de l''étape e) avec 1 équivalent ou plus d'un acide tel que défini ci-dessus. A titre d'exemple, dans le cas d'utilisation d'oligoalginate comme matière première, la composition molaire est d'environ : *n*-alkyl α-D-mannopyranosiduronique 30% ; *n*-alkyl α,β-D-mannofuranosiduronique 15 %; *n*-alkyl α,β-L-gulopyranosiduronique 25% ; *n*-alkyl α,β-L-gulofuranosidurono-6,3-lactone 30%. A titre d'exemple, dans le cas d'utilisation d'alginate semi-raffiné issu de *Laminaria digitata* comme matière première, la composition massique est d'environ : dérivés *n*-alkyl D-mannuronique et L-guluronique 45%, *n-*alkyl L-fucose 20%, *n*-alkyl α,β-D-glucopyranose 35%.

La présente invention a également pour objet une composition obtenue par un procédé selon l'invention. De préférence, ladite composition associe sous forme de monomères des dérivés de l'acide L-guluronique ou des deux acides uroniques (acide L-guluronique et acide D-mannuronique) issus du même polysaccharide, et dans certains cas des hexoses et pentoses dérivés d'autres polysaccharides présents dans l'extrait d'algues (cellulose, hemicellulose, laminaranes, fucane : cas des alginates semi-raffinés).

La présente invention a également pour objet l'utilisation d'une composition selon la présente invention comme agent tensioactif. De préférence, ledit agent tensioactif est choisi des agents solubilisants, hydrotropes, mouillants, moussants, émulsionnants, émulsifiants et/ou détergents.

La présente invention a également pour objet un tensioactif comprenant une composition selon l'invention. Ledit tensioactif peut présenter les propriétés suivantes :

| Nombre d'atomes de carbone de la chaîne lipophile (alkyle) : | Tensioactif à deux chaînes lipophiles : alkyl mannoside uronates d'alkyle et/ou alkyl guloside uronates d'alkyle |
|---|---|
| Entre 1 et 6 | Agents hydrotropes et/ou solubilisants |
| Entre 6 et 14 | Agents émulsifiants huile dans eau (H/E) |
| Entre 16 et 22 | Agents émulsionnants eau dans huile (E/H) |

| Nombre d'atomes de carbone de la chaîne lipophile (alkyle) : | Tensioactif à une chaîne lipophile : | |
|---|---|---|
| | sels des acides alkyl mannoside uroniques et/ou acides alkyl guloside uroniques | acides alkyl mannoside uroniques et/ou acides alkyl guloside uroniques |
| Entre 4 et 6 | Agents solubilisants et/ou hydrotropes | |
| Entre 6 et 10 | Agents mouillants et/ou détergents | |
| Entre 10 et 16 | Agents moussants et/ou détergents | |
| Entre 16 et 22 | Agents émulsionnants | |

Le procédé et les compositions de la présente invention répondent au principe de la « chimie bleue » :
- en utilisant exclusivement des matières premières biosourcées (alginates (bactériens, raffinés ou semi-raffinés), oligoalginates, poly(oligo)guluronates, alcools gras ou non) ou biocompatibles / biodégradables ;
- en mettant en œuvre une méthodologie qui permet de maîtriser la réactivité à la fois de l'acide L-guluronique et de l'acide D-mannuronique issus des poly(oligo)guluronates, des oligoalginates, des alginates bactériens, raffinés ou semi-raffinés, de façon à exploiter l'acide L-guluronique ou le mélange des deux saccharides (acide L-guluronique et acide D-mannuronique) pour l'obtention des compositions tensioactives correspondantes ;
- en utilisant des conditions qui autorisent et valorisent la présence d'autres polysaccharides (notamment cellulose, hemicelluloses, laminaranes, fucanes) en plus de l'alginate (e.g. alginates semi-raffinés), conduisant ainsi à des compositions associant à la fois des dérivés uroniques (acide L-guluronique et acide D-mannuronique), des hexoses (notamment glucose, fucose) et des pentoses (notamment xylose);
- en proposant des conditions réactionnelles sans solvants organiques autres que les alcools réactifs, ne produisant pas de déchets (recyclage des alcools à chaines courtes (n-butanol, etc.)) et utilisant des réactifs biodégradables (acide méthanesulfonique et analogues) ;
- en réalisant l'ensemble des réactions selon un procédé « one pot » sans isolement, ni purification des intermédiaires réactionnels ;
- en utilisant des conditions simples de purification partielle des bruts réactionnels (élimination des sels et/ou des alcools gras résiduels) ou d'isolement des compositions tensioactives qui permettent d'aboutir aux composés dérivés et aux compositions présentant des performances/caractéristiques en adéquation avec les besoins du marché

Ainsi la présente invention permet à la fois de réduire les coûts de production des compositions tensioactives et de proposer de nouvelles compositions entièrement bio-sourcées (algues brunes, alginates (bactériens, raffinés ou semi-raffinés), oligoalginates, poly(oligo)guluronates) ou biocompatibles / biodégradables, à faible écotoxicité, à partir de ressources végétales d'origine marine, dans un objectif d'amélioration de leurs performances.

Le procédé qui fait l'objet de la présente invention permet de produire des compositions dérivées de l'acide L-guluronique ou dérivées à la fois de l'acide L-guluronique et de l'acide D-mannuronique et pouvant comporter des tensioactifs non-ioniques dérivés de l'hexose (notamment glucose, fucose) et de pentoses (notamment xylose). Ces compositions présentent de très bonnes propriétés tensioactives pouvant trouver des applications notamment dans des domaines tels que les agents solubilisants, mouillants, détergents, moussants, émulsionnants. En particulier la possibilité d'accéder à des compositions constituées d'un mélange de tensioactifs anioniques et non-ioniques à partir de la même matière première (e.g. alginates semi-raffinés) et selon un procédé « one-pot », constitue une alternative importante aux formulations à base de tensioactifs anioniques et non-ioniques produits de façon indépendante les uns des autres et provenant de matières premières de nature différente. En effet, Les procédés « one-pot » permettent d'accéder à la fois à des tensioactifs non-ioniques et anioniques directement à partir des oligomères (oligoalginates) constitués des deux acides uroniques, des polymères entiers (alginates) sous forme raffiné ou semi-raffiné, ou bien des algues brunes. Ils évitent le recours à des formes monosaccharides comme matières premières, ce qui simplifie largement le procédé et diminue le coût des compositions tensioactives.

D'autres avantages pourront encore apparaître à l'homme du métier à la lecture des exemples ci-dessous, illustrés par les figures annexées, donnés à titre illustratif.

### Brève description des figures

- La figure 1 représente les mesures tensiométriques effectuées sur les dérivés C12-C12 issus de poly(oligo)guluronate, d'oligoalginate et d'alginate semi-raffiné.
- La figure 2 représente les mesures tensiométriques effectuées sur les dérivés Na-C12 issus de poly(oligo)guluronate, d'oligoalginate et d'alginate semi-raffiné.
- La figure 3 représente les mesures tensiométriques effectuées sur les dérivés Na-C18:1 issus d'alginate semi-raffiné.
- La figure 4 représente les mesures tensiométriques effectuées sur les dérivés H-C12 issus de poly(oligo)guluronate, d'oligoalginate et d'alginate semi-raffiné.
- La figure 5 représente le procédé de préparation de compositions comprenant des alkyl guloside uronates d'alkyle de l'invention à partir de différentes matières premières algales, et des procédés de préparation de l'art antérieur.

### EXEMPLES

### EXEMPLE 1 : PRÉPARATION DE COMPOSITION COMPRENANT DES ALKYL GULOSIDE URONATES D'ALKYLE À PARTIR DE POLY(OLIGO)GULURONATES

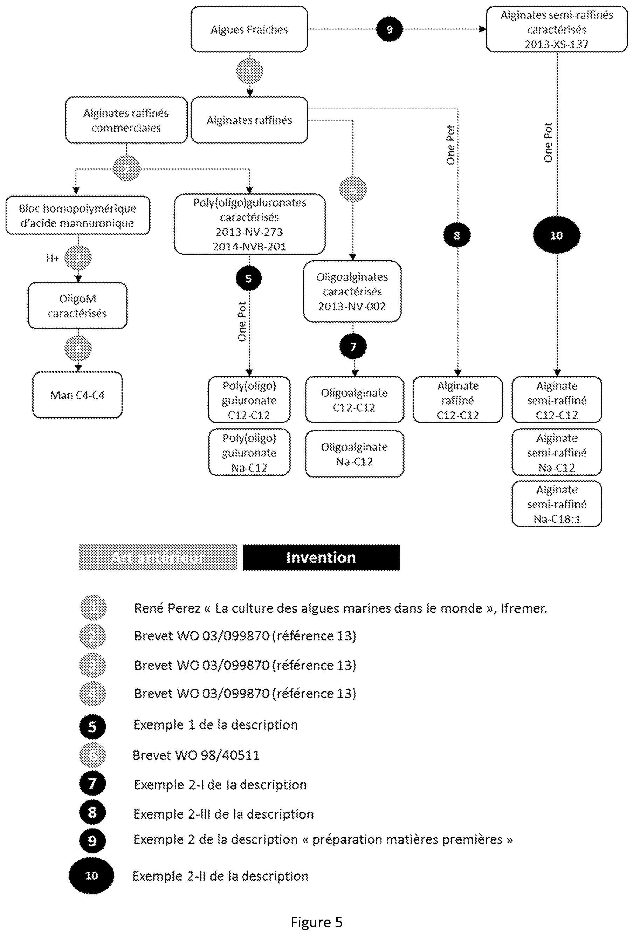

### Poly(oligo)guluronate C12-C12 (figure 5)

2013-NV-273: poly(oligo)guluronate obtenu selon procédé de la Demande Internationale WO 03/099870 [13]

| **Libellé** | **Méthode** | **Résultat** | **Unités** |
|---|---|---|---|
| **Matière sèche** | Masse constante à 103°C | 83,9 | %sec/brut |
| **Matière minérale** | 12 h, 550°C | 25,0 | %sec/brut |
| **Rapport (M/G)** | Par calcul, RMN du proton | 0,2 | |
| **DP** | Par calcul | 21,5 | |

Le poly(oligo)guluronate 2013-NV-273 (500 mg, 1,79 mmol CO₂⁻, 1 éq) a été dispersé dans l'eau (0,9 mL) et le butanol (25 mL, 273 mmol, 153 éq). La solution d'acide méthanesulfonique 70% (401 µL, 3,94 mmol, 2,2 éq) a été ajoutée et le mélange a été chauffé à reflux sous vive agitation. L'eau formée dans le milieu a progressivement été éliminée par distillation azéotropique. A 7 h de réaction, le mélange a été refroidi à température ambiante.

Le dodécanol (1,6 mL, 7,2 mmol, 4 éq) et la solution d'acide méthanesulfonique 70% (182 µL, 1,79 mmol, 1 éq) ont été ajoutés. Le mélange a été agité à 70°C sous pression réduite (jusqu'à 5 mbar).

Une fois le butanol complètement éliminé (1,25 h), le mélange a été neutralisé par l'ajout de NaOH 1 M (4,5 mL) et d'eau (20 mL) à température ambiante et pression atmosphérique. L'ensemble a été chauffé à 80°C sous vive agitation pendant 15 min. Une fois le mélange revenu à température ambiante, la phase aqueuse a été séparée de la phase organique. Cette dernière a enfin été séchée par distillation azéotropique de l'eau à l'aide de butanol. L'excès de dodécanol présent dans le brut organique a pu être éliminé partiellement ou totalement par distillation moléculaire.

Après une éventuelle purification par chromatographie sur gel de silice (CH₂Cl₂/MeOH 97:3), un mélange de produits a été obtenu (270 mg), dont la composition molaire est : *n*-dodécyl α-D-mannopyranosiduronate de dodécyle 9% ; *n*-dodécyl α-D-mannofuranosiduronate de dodécyle 2% ; *n-*dodécyl β-D-mannofuranosidurono-6,3-lactone 5% ; *n*-dodécyl α-L-gulopyranosiduronate de dodécyle 15% ; *n*-dodécyl β-L-gulopyranosiduronate de dodécyle 25% ; *n*-dodécyl β-L-gulofuranosiduronate de dodécyle 12% ; *n*-dodécyl α-L-gulofuranosidurono-6,3-lactone 21%; *n*-dodécyl β-L-guiofuranosidurono-6,3-lactone 11%.

### Poly(oligo)guluronate Na-C12 (figure 5)

2014-NVR-201 : poly(oligo)guluronate obtenu selon procédé de la Demande Internationale WO 03/099870 [13]

| **Libellé** | **Méthode** | **Résultat** | **Unités** |
|---|---|---|---|
| **Matière sèche** | Masse constante à 103°C | 100,0 | %sec/brut |
| **Matière minérale** | 12 h, 550°C | 26,3 | %sec/brut |
| **Rapport (M/G)** | Par calcul, RMN du proton | 0,039 | |
| **DP** | Par calcul | 30 | |

Le poly(oligo)guluronate 2014-NVR-201 (1,000 g, 4,2 mmol CO₂⁻, 1 éq) a été dispersé dans l'eau (3,0 mL) et le butanol (58 mL, 634 mmol, 151 éq). La solution d'acide méthanesulfonique 70% (940 µL, 9,24 mmol, 2,2 éq) a été ajoutée et le mélange a été chauffé à reflux sous vive agitation. L'eau formée dans le milieu a été progressivement éliminée par distillation azéotropique. A 7 h de réaction, le mélange a été refroidi à température ambiante.

Le dodécanol (3,7 mL, 16,6 mmol, 4 éq) et la solution d'acide méthanesulfonique 70% (423 µL, 4,16 mmol, 1 éq) ont été ajoutés. Le mélange a été agité à 70°C sous pression réduite (jusqu'à 5 mbar).

Une fois le butanol complètement éliminé (1,25 h), une solution de NaOH 0,4 M (37 mL) a été ajoutée et le mélange a été laissé sous vive agitation à 70°C pendant 1 h. L'eau a ensuite été éliminée par lyophilisation ou par distillation azéotropique avec du butanol. L'excès de dodécanol présent dans le produit brut a pu être éliminé partiellement ou totalement par extraction solide-liquide avec de l'acétone.

A l'issu de ce traitement, un mélange de produits a été obtenu (2,981 g), dont le pourcentage de matière minérale est de 38,5 %/brut.

### Poly(oligo)guluronate H-C12

Une partie de ce mélange de produits ci-dessus (816 mg) a été dissout dans de l'eau glacée (15 mL) puis une solution d'acide chlorhydrique 1 M (2,0 mL) a été ajoutée. La solution aqueuse a été extraite à l'acétate d'éthyle (3 x 10 mL). Les phases organiques ont été rassemblées et lavées avec une solution de NaCl saturée (20 mL). La phase organique a été séchée avec du MgSO₄ puis concentrée sous vide. Un mélange de produits a été obtenu (195 mg), dont la composition molaire est : *n*-dodécyl α-D-mannopyranosiduronique 21 % ; *n*-dodécyl β-L-gulopyranosiduronique 11 % ; *n*-dodécyl α-L-gulofuranosidurono-6,3-lactone 34 % ; *n*-dodécyl β-L-gulofuranosidurono-6,3-lactone 34 %.

### EXEMPLE 2 : PRÉPARATION DE COMPOSITION COMPRENANT UN MÉLANGE D'ALKYL GULOSIDE URONATES D'ALKYLE ET D'ALKYL MANNOSIDE URONATES D'ALKYLE

Préparation des matières premières : Les procédés d'extraction des alginates sont classiquement utilisés au CEVA (René Perez « La culture des algues marines dans le monde », Ifremer : voir figure 5). Ils mettent en jeu une lixiviation acide d'algues fraîches ou sèches (lavage à l'eau de mer des algues récoltées, dépigmentation dans le formol, broyage, extraction avec de l'acide sulfurique à 0,2N à température ambiante, égouttage et rinçage des algues lixiviées avec de l'eau distillée), suivi d'une solubilisation des alginates de sodium par augmentation du pH du milieu puis d'une séparation solide/liquide afin d'éliminer les résidus algaux (addition d'une solution de Na₂CO₃ à 1,5% à 50g sec de matière algale lixiviée selon un ratio algue sèche/solution Na₂CO₃ à 1,5% de 0,025, agitation au réacteur IKA pendant 3h à 55°C, refroidissement dans un bain d'eau avec quelques glaçons pour éviter des écarts de température trop important, centrifugation 5 minutes à 6000t/min, séparation solide/liquide). A ce stade, la fraction liquide peut être congelée et lyophilisée et constitue les alginates semi-raffinés sous forme d'alginates de sodium. Afin d'obtenir des alginates raffinés, une purification est apportée aux étapes précédentes. Après séparation des résidus algaux, cette dernière étape de purification consiste en une précipitation de l'acide alginique par abaissement du pH, suivie de plusieurs lavages à l'eau acide afin d'éliminer les co-produits. Une augmentation du pH avec Na₂CO₃ permet de nouveau de solubiliser les alginates de sodium en limitant les sels, par rapport à l'utilisation de la soude. Enfin, une étape finale de congélation puis lyophilisation permet d'arriver au produit final. Afin d'obtenir les oligoalginates, saturés ou insaturés, la solution d'alginate est traitée par voie enzymatique ou acide afin d'abaisser le degré de polymérisation des alginates de 20 à 3.

### I- à partir d'oligoalginates

### Oligoalginate C12-C12 (figure 5)

2013-NV-002 : oligoalginate obtenu selon le procédé décrit dans l'exemple 2 et/ou obtenu selon procédé de la Demande Internationale WO 98/40511 [15].

| **Libellé** | **Méthode** | **Résultat** | **Unités** |
|---|---|---|---|
| **Matière sèche** | Masse constante à 103°C | 90,1 | %sec/brut |
| **Matière minérale** | 12 h, 550°C | 44,3 | %sec/sec |
| **Rapport (M/G)** | Par calcul, RMN du proton | 1,4 | |
| **DP** | Par calcul | 12,7 | |

L'oligoalginate 2013-NV-002 (500 mg, 1,42 mmol CO₂⁻, 1 éq) a été dispersé dans l'eau (0,9 mL) et le butanol (19,5 mL, 213 mmol, 150 éq). La solution d'acide méthanesulfonique 70% (318 µL, 3,12 mmol, 2,2 éq) a été ajoutée et le mélange a été chauffé à reflux sous vive agitation. L'eau formée dans le milieu a été progressivement éliminée par distillation azéotropique. A 7 h de réaction, le mélange a été refroidi à température ambiante.

Le dodécanol (1,3 mL, 5,8 mmol, 4 éq) et la solution d'acide méthanesulfonique 70% (145 µL, 1,43 mmol, 1 éq) ont été ajoutés. Le mélange a été agité à 70°C sous pression réduite (jusqu'à 5 mbar).

Une fois le butanol complètement éliminé (1,25 h), le mélange a été neutralisé par l'ajout de NaOH 1 M (3,2 mL) et d'eau (20 mL) à température ambiante et pression atmosphérique. L'ensemble a été chauffé à 80°C sous vive agitation pendant 15 min. Une fois le mélange revenu à température ambiante, la phase aqueuse a été séparée de la phase organique. Cette dernière a enfin été séchée par distillation azéotropique de l'eau à l'aide de butanol. L'excès de dodécanol présent dans le brut organique a pu être éliminé partiellement ou totalement par distillation moléculaire.

Après une éventuelle purification par chromatographie sur gel de silice (CH₂Cl₂/MeOH 97:3), un mélange de produits a été obtenu (231 mg), dont la composition molaire est : *n*-dodécyl α-D-mannopyranosiduronate de dodécyle 29% ; *n*-dodécyl α-D-mannofuranosiduronate de dodécyle 6% ; *n-*dodécyl a,β-D-mannofuranosidurono-6,3-lactone 12%; n-dodécyl α,β-L-gulopyranosiduronate de dodécyle 23% ; n-dodécyl β-L-gulofuranosiduronate de dodécyle 9% ; n-dodécyl α,β-L-gulofuranosidurono-6,3-lactone 21%.

### Oligoalginate Na-C12 (figure 5)

L'oligoalginate 2013-NV-002 (1,000 g, 2,84 mmol CO₂⁻, 1 éq) a été dispersé dans l'eau (2,0 mL) et le butanol (39 mL, 426 mmol, 150 éq). La solution d'acide méthanesulfonique 70% (634 µL, 6,24 mmol, 2,2 éq) a été ajoutée et le mélange a été chauffé à reflux sous vive agitation. L'eau formée dans le milieu a été progressivement éliminée par distillation azéotropique. A 7 h de réaction, le mélange a été refroidi à température ambiante.

Le dodécanol (2,5 mL, 11,2 mmol, 4 éq) et la solution d'acide méthanesulfonique 70% (290 µL, 2,85 mmol, 1 éq) ont été ajoutés. Le mélange a été agité à 70°C sous pression réduite (jusqu'à 5 mbar).

Une fois le butanol complètement éliminé (1,25 h), une solution de NaOH 0,4 M (25 mL) a été ajoutée et le mélange a été laissé sous vive agitation à 70°C pendant 1 h. L'eau a ensuite été éliminée par lyophilisation ou par distillation azéotropique avec du butanol. L'excès de dodécanol présent dans le produit brut a pu être éliminé partiellement ou totalement par extraction solide-liquide avec de l'acétone.

A l'issu de ce traitement, un mélange de produits a été obtenu (2,532 g), dont le pourcentage de matière minérale est de 41,8 %/brut.

### Oligoalginate H-C12

Une partie de ce mélange de produits ci-dessus (837 mg) a été dissout dans de l'eau glacée (15 mL) puis une solution d'acide chlorhydrique 1 M (2,0 mL) a été ajoutée. La solution aqueuse a été extraite à l'acétate d'éthyle (3 x 10 mL). Les phases organiques ont été rassemblées et lavées avec une solution de NaCl saturée (20 mL). La phase organique a été séchée avec MgSO₄ puis concentrée sous vide. Un mélange de produits a été obtenu (251 mg), dont la composition molaire est : *n*-dodécyl α-D-mannopyranosiduronique 32 % ; n-dodécyl β-L-gulopyranosiduronique 13 % ; *n*-dodécyl α-L-gulofuranosidurono-6,3-lactone 29 % ; *n*-dodécyl β-L-gulofuranosidurono-6,3-lactone 26 %.

### II- à partir d'alginate semi-raffiné

### Alginate semi-raffiné C12-C12 (figure 5)

2013-XS-137 : alginate semi-raffiné issu de *Laminaria digitata* obtenu selon le procédé décrit dans l'exemple 2.

| **Libellé** | **Méthode** | **Résultat** | **Unités** |
|---|---|---|---|
| **Matière sèche** | Masse constante à 103°C | 94,9 | %sec/brut |
| **Matière minérale** | 12 h, 550°C | 47,0 | %sec/sec |
| **Teneur en acide mannuronique et guluronique** | Méthanolyse | 29,2 | %sec/sec |
| **Teneur en glucose** | Méthanolyse | 10,9 | %sec/sec |
| **Teneur en xylose** | Méthanolyse | < 0,5 | %sec/sec |
| **Teneur en fucose** | Méthanolyse | 2,1 | %sec/sec |
| **Rapport (M/G)** | Par calcul, RMN du proton | 2,6 | |

L'alginate semi-raffiné 2013-XS-137 issu de *Laminaria digitata* (1,000 g, 2,15 mmol unités sucre, 1 éq) a été dispersé dans l'eau (30 mL) et la solution d'acide méthanesulfonique 70% (1,09 mL, 10,7 mmol, 5 éq) a été ajoutée. Le mélange a été chauffé à reflux sous vive agitation. A 8 h de réaction, le butanol (30 mL, 328 mmol, 153 éq) a été ajouté et le mélange a été laissé à reflux sous vive agitation. L'eau présente dans le milieu a été progressivement éliminée par distillation azéotropique.

Après 15 h de réaction supplémentaires, et une fois le mélange revenu à température ambiante, le dodécanol (1,92 mL, 8,6 mmol, 4 éq) a été ajouté. Le mélange a été agité à 70°C sous pression réduite (jusqu'à 5 mbar).

Une fois le butanol complètement éliminé (1,25 h), le mélange a été neutralisé par l'ajout de NaOH 1 M (3,55 mL) et d'eau (30 mL) à température ambiante et pression atmosphérique. L'ensemble a été chauffé à 80°C sous vive agitation pendant 15 min. Une fois le mélange revenu à température ambiante, la phase aqueuse a été séparée de la phase organique. Cette dernière a ensuite été lavée à l'eau (30 mL) par agitation à 80°C pendant 15 min. La phase organique a été récupérée puis séchée par distillation azéotropique de l'eau à l'aide de butanol. L'excès de dodécanol présent dans le produit brut a pu être éliminé partiellement ou totalement par distillation moléculaire.

Après une éventuelle purification par chromatographie sur gel de silice (CH₂Cl₂/MeOH 97:3 ensuite 96:4 puis 90:10), un mélange de produits a été obtenu (377 mg), dont la composition massique est : *n-*dodécyl α-D-mannofuranosiduronate de dodécyle, *n*-dodécyl α,β-D-mannofuranosidurono-6,3-lactone, *n*-dodécyl β-L-gulofuranosiduronate de dodécyle et *n*-dodécyl α,β-L-guiofuranosidurono-6,3-lactone 35%, *n-*dodécyl α-D-mannopyranosiduronate de dodécyle et *n*-dodécyl α,β-L-gulopyranosiduronate de dodécyle 25%, *n*-dodécyl L-fucose 14%, *n-*dodécyl α,β-D-glucopyranose 26%.

### Alginate semi-raffiné Na-C12 (figure 5)

L'alginate semi-raffiné 2013-XS-137 issu de *Laminaria digitata* (2,000 g, 4,3 mmol unités sucre, 1 éq) a été dispersé dans l'eau (60 mL) et la solution d'acide méthanesulfonique 70% (2,19 mL, 21,5 mmol, 5 éq) a été ajoutée. Le mélange a été chauffé à reflux sous vive agitation. A 8 h de réaction, le butanol (60 mL, 656 mmol, 152 éq) a été ajoutée et le mélange a été laissé à reflux sous vive agitation. L'eau présente dans le milieu a été progressivement éliminée par distillation azéotropique.

Après 15 h de réaction supplémentaires, et une fois le mélange revenu à température ambiante, le dodécanol (3,8 mL, 17 mmol, 4 éq) a été ajouté. Le mélange a été agité à 70°C sous pression réduite (jusqu'à 5 mbar).

Une fois le butanol complètement éliminé (1,25 h), une solution de NaOH 0,2 M (60 mL) a été ajoutée et le mélange a été laissé sous vive agitation à 70°C pendant 1 h. L'eau a ensuite été éliminée par lyophilisation ou par distillation azéotropique avec du butanol. L'excès de dodécanol présent dans le produit brut a pu être éliminé partiellement ou totalement par extraction solide-liquide avec de l'acétone.

A l'issu de ce traitement, un mélange de produits a été obtenu (3,592 g), dont le pourcentage de matière minérale est de 42,4 %/brut.

### Alginate semi-raffiné H-C12

Une partie de ce mélange de produits ci-dessus (1,081 g) a été dissout dans de l'eau glacée (15 mL) puis une solution d'acide chlorhydrique 1 M (2,0 mL) a été ajoutée. La solution aqueuse a été extraite à l'acétate d'éthyle (3 x 10 mL). Les phases organiques ont été rassemblées et lavées avec une solution de NaCl saturée (20 mL). La phase organique a été séchée avec MgSO₄ puis concentrée sous vide. Un mélange de produits a été obtenu (138 mg), dont la composition massique est : dérivés *n*-dodécyl D-mannuronique et L-guluronique 67 %, *n*-dodécyl L-fucose et *n*-dodécyl α,β-D-glucopyranose 33 %.

### Alginate semi-raffiné Na-C18:1 (figure 5)

L'alginate semi-raffiné 2013-XS-137 issu de *Laminaria digitata* (2,000 g, 4,3 mmol unités sucre, 1 éq) a été dispersé dans l'eau (60 mL) et la solution d'acide méthanesulfonique 70% (2,19 mL, 21,5 mmol, 5 éq) a été ajoutée. Le mélange a été chauffé à reflux sous vive agitation. A 8 h de réaction, le butanol (60 mL, 656 mmol, 152 éq) a été ajouté et le mélange a été laissé à reflux sous vive agitation. L'eau présente dans le milieu a été progressivement éliminée par distillation azéotropique. Après 15 h de réaction supplémentaires, et une fois le mélange revenu à température ambiante, l'alcool oléïque (5,4 mL, 17 mmol, 4 éq) a été ajouté. Le mélange a été agité à 70°C sous pression réduite (jusqu'à 5 mbar). Une fois le butanol complètement éliminé (1,25 h), une solution de NaOH 0,2 M (60 mL) a été ajoutée et le mélange a été laissé sous vive agitation à 70°C pendant 1 h. L'eau a ensuite été éliminée par lyophilisation ou par distillation azéotropique avec du butanol. L'excès d'alcool oléïque présent dans le produit brut a pu être éliminé partiellement ou totalement par extraction solide-liquide avec de l'acétone. A l'issu de ce traitement, un mélange de produits a été obtenu (3,418 g), dont le pourcentage de matière minérale est de 44,1 %/brut.

### III- à partir d'alginate raffiné

### Alginate raffiné C12-C12 (figure 5)

2013-NV-277 : alginate raffiné issu de *Laminaria digitata* obtenu selon le procédé décrit dans l'exemple 2.

| **Libellé** | **Méthode** | **Résultat** | **Unités** |
|---|---|---|---|
| **Matière sèche** | Masse constante à 103°C | 94,2 | %sec/brut |
| **Matière minérale** | 12 h, 550°C | 37,2 | %sec/sec |
| **Rapport (M/G)** | Par calcul, RMN du proton | 2,6 | |
| **DP** | Par calcul | 362 | |

L'alginate raffiné 2013-NV-277 issu de *Laminaria digitata* (1,000 g, 2,38 mmol CO₂⁻, 1 éq) a été dispersé dans l'eau (30 mL) et la solution d'acide méthanesulfonique 70% (1,21 mL, 11,9 mmol, 5 éq) a été ajoutée. Le mélange a été chauffé à reflux sous vive agitation. A 8 h de réaction, le butanol (33 mL, 361 mmol, 152 éq) a été ajouté et le mélange a été laissé à reflux sous vive agitation. L'eau présente dans le milieu a été progressivement éliminée par distillation azéotropique. Après 15 h de réaction supplémentaires, et une fois le mélange revenu à température ambiante, le dodécanol (2,12 mL, 9,5 mmol, 4 éq) a été ajouté. Le mélange a été agité à 70°C sous pression réduite (jusqu'à 5 mbar). Une fois le butanol complètement éliminé (1,25 h), le mélange a été neutralisé par l'ajout de NaOH 1 M (3,95 mL) et d'eau (30 mL) à température ambiante et pression atmosphérique. L'ensemble a été chauffé à 80°C sous vive agitation pendant 15 min. Une fois le mélange revenu à température ambiante, la phase aqueuse a été séparée de la phase organique. Cette dernière a enfin été séchée par distillation azéotropique de l'eau à l'aide de butanol. L'excès de dodécanol présent dans le brut organique a pu être éliminé partiellement ou totalement par distillation moléculaire. Après une éventuelle purification par chromatographie sur gel de silice (CH₂Cl₂/MeOH 97:3), un mélange de produits a été obtenu (387 mg), dont la composition molaire est : *n*-dodécyl α-D-mannofuranosiduronate de dodécyle 8%, *n*-dodécyl β-D-mannofuranosidurono-6,3-lactone 4%, *n*-dodécyl β-L-gulofuranosiduronate de dodécyle 11%, *n*-dodécyl α-L-gulofuranosidurono-6,3-lactone 4%, *n-*dodécyl β-L-gulofuranosidurono-6,3-lactone 7%, *n*-dodécyl α-D-mannopyranosiduronate de dodécyle 48%, *n*-dodécyl α-L-gulopyranosiduronate de dodécyle 5%, *n*-dodécyl β-L-gulopyranosiduronate de dodécyle 13%.

### EXEMPLE 3 : MESURES TENSIOMÉTRIQUES DES COMPOSITIONS DE L'INVENTION

### Tension interfaciale

Les mesures de tension interfaciale ont été réalisées à l'aide d'un tensiomètre Krüss avec un anneau en platine suspendu horizontalement. Avant chaque mesure, l'anneau a été minutieusement nettoyé et séché à la flamme. Le godet pour échantillon est un récipient cylindrique en verre placé dans une enceinte thermorégulée. Les solutions d'échantillon ont été préparées avec de l'huile de tournesol. Les mesures de tension interfaciale ont été réalisées entre de l'eau Milli-Q et les solutions d'échantillon dans l'huile.

Les différents lots de tensioactifs dérivés C12-C12 obtenus à l'issue de la purification par chromatographie ont été caractérisés par des mesures de tension interfaciale entre de l'eau et de l'huile de tournesol.

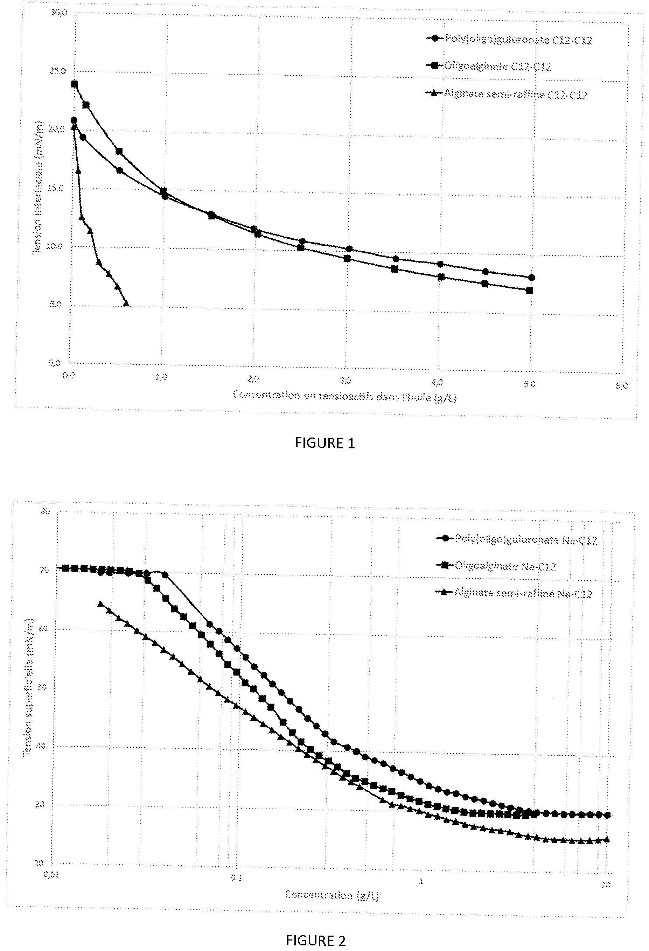

Les résultats représentés sur la Figure 1 montrent que la tension interfaciale est bien diminuée quel que soit le lot de tensioactifs. Cependant, le lot obtenu à partir d'alginate semi-raffiné est celui qui permet de diminuer le plus efficacement la tension. En effet, il permet d'atteindre les mêmes valeurs de tension à des concentrations 10 fois plus faibles. Par exemple, 7,0 mN/m à 0,5 g/L au lieu de 5,0 g/L.

### Tension superficielle

Les mesures de tension superficielle ont été réalisées à l'aide d'un tensiomètre Krüss avec un anneau en platine suspendu horizontalement. Avant chaque mesure, l'anneau a été minutieusement nettoyé et séché à la flamme. Le godet pour échantillon est un récipient conique en PTFE placé dans une enceinte thermorégulée. Les solutions d'échantillon ont été préparées avec de l'eau Milli-Q et continuellement agitées à l'aide d'un barreau magnétique avant chaque mesure.

Les différents lots de tensioactifs dérivés Na-C12 obtenus à l'issue de l'extraction solide-liquide ont été caractérisés par des mesures de tension superficielle.

Les résultats représentés sur la Figure 2 montrent tout d'abord que tous les lots de tensioactifs permettent de diminuer efficacement la tension superficielle de l'eau puisque des valeurs entre 25 et 30 mN/m ont été mesurées au niveau des concentrations micellaires critiques (CMC) respectives. Cependant, des valeurs de CMC très différentes sont obtenues suivant les lots. En effet, le lot issu d'oligoalginate présente la plus faible valeur (1,9 g/L) alors que celui obtenu à partir de poly(oligo)guluronate présente la valeur la plus élevée (5,0 g/L). Le lot issu d'alginate semi-raffiné (*L. digitata*) présente une valeur de CMC intermédiaire : 4,7 g/L. Il est cependant possible de constater que ce dernier est celui qui permet d'abaisser le plus efficacement la tension superficielle, puisqu'une tension de 25,7 mN/m a été mesurée au niveau de la CMC. Il est également important de noter que pour toutes les valeurs de concentration, les plus faibles valeurs de tension superficielle ont été mesurées avec le lot obtenu à partir d'alginate semi-raffiné.

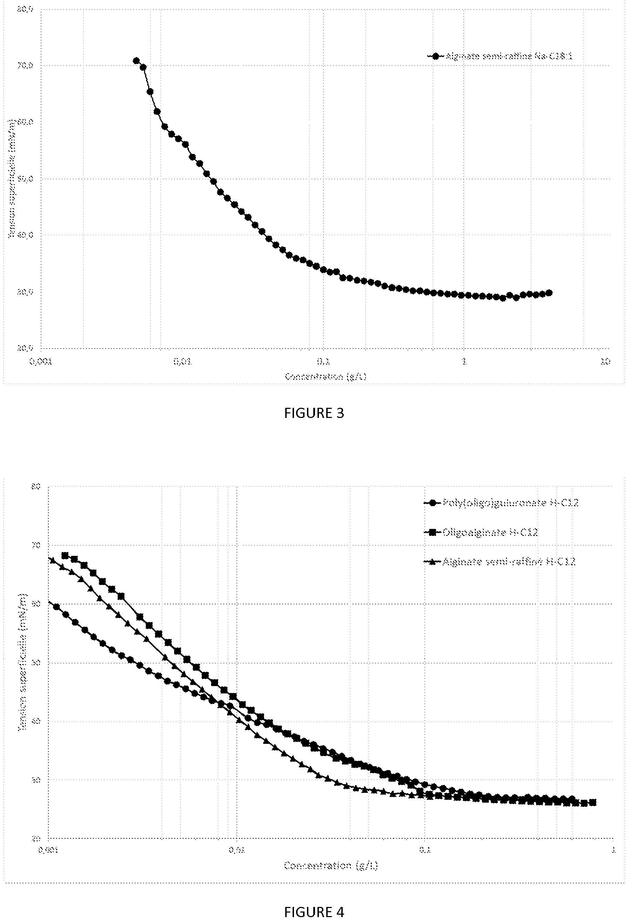

Le lot de tensioactifs dérivés Na-C18:1 issu d'alginate semi-raffiné obtenu à l'issue de l'extraction solide-liquide a été caractérisé par des mesures de tension superficielle (Figure 3).

Les résultats représentés sur la Figure 3 montrent que le lot permet de diminuer efficacement la tension superficielle. Une CMC de 0,55 g/L et une γ_{CMC} de 30 mN/m ont été mesurées.

Les différents lots de tensioactifs dérivés H-C12 obtenus après acidification des dérivés Na-C12 et élimination des sels inorganiques ont été caractérisés par des mesures de tension superficielle (Figure 4).

Les résultats représentés sur la Figure 4 montrent tout d'abord que tous les lots de tensioactifs permettent de diminuer efficacement la tension superficielle de l'eau puisque des valeurs inférieures à 30 mN/m ont été mesurées au niveau des CMC. Le lot obtenu à partir de poly(oligo)guluronate présente une CMC de 0,12 g/L avec une γ_{CMC} de 28,8 mN/m. Les résultats obtenus avec le lot issu d'oligoalginate sont légèrement meilleurs puisque la CMC est de 0,11 g/L et la γ_{CMC} de 27,6 mN/m. Enfin, le lot obtenu à partir d'alginate semi-raffiné (*L. digitata*) permet d'obtenir la CMC la plus faible avec une valeur de 0,04 g/L et une γ_{CMC} de 29 mN/m. Il est également important de noter que pour les concentrations inférieures à 0,008 g/L, les plus faibles valeurs de tension superficielle ont été mesurées avec le lot obtenu à partir de poly(oligo)guluronate.

### Listes des références

1- Sugar-based Surfactants : fundamentals and applications », Surfactant science series vol.143, Ed. C. Carnero Ruiz, CRC Press Taylor & Francis Group, 2009 (ISBN 978-1-4200-5166-7)
2- Behler et al., in Proceedings 6th World Surfactant Congress, CESIO June, Berlin, 2004
3- Brevet EP 0532370
4- Brevet US 5,312,907
5- Demande de Brevet FR 2717177
6- Demande internationale WO 93/02092
7- Demande internationale WO 98/12228
8- Brevet US 5,147,861
9- Benvegnu et al., Topics in Current Chemistry, 294 : 143-164, 2010
10- Roussel et al., Eur. J. Org. Chem., 3085-3094, 2005
11- Demande de Brevet FR 02/840306
12- Demande internationale WO 03/104248
13- Demande internationale WO 03/099870
14- Demande internationale WO 09/134368
15- Demande internationale WO 98/40511

## Revendications

1. Procédé de préparation d'une composition comprenant :
(i) des alkyl guloside uronates d'alkyle de formules (Ia), (Ib) et (Ic) : 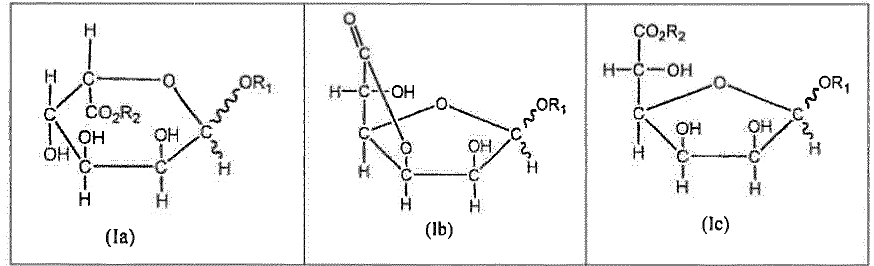 ou
(ii) un mélange d'alkyl guloside uronates d'alkyle de formule (Ia, Ib et Ic) et d'alkyl mannoside uronates d'alkyle de formule (IIa), (IIb) et (IIc) : 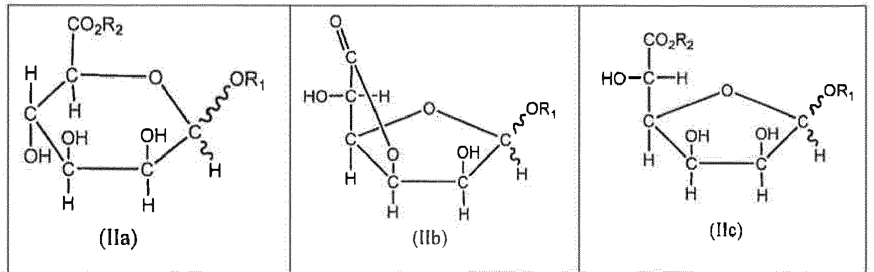 où
- R₁ est une chaîne alkyle de 2 à 22 atomes de carbone, linéaire ou ramifiée, saturée ou insaturée ;
- R₂ est un atome d'hydrogène, R₁, un atome de métal alcalin, un atome de métal alcalino-terreux, ou un groupe ammonium quaternaire de formule (III) : 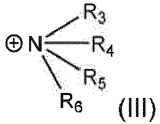
- où chacun des R₃ à R₆ est indépendamment un atome d'hydrogène, un alkyle ayant de 1 à 6 atomes de carbone ou un hydroxyalkyle ayant de 1 à 6 atomes de carbone et **caractérisé en ce que** ledit procédé comprend :
a) une étape d'hydrolyse de poly(oligo)guluronates, d'oligoalginates et/ou d'alginates ;
b) une étape d'estérification et glycosylation de l'hydrolysat issu de l'étape a) par un alcool de formule ROH, linéaire ou ramifié, saturé ou insaturé, ayant de 1 à 4 atomes de carbone ;
c) une étape de trans-estérification et trans-glycosylation du milieu réactionnel issu de l'étape b) par un alcool de formule R'OH, linéaire ou ramifié, saturé ou insaturé, ayant de 2 à 22 atomes de carbone ;
d) éventuellement une étape de neutralisation du milieu réactionnel issu de l'étape c) en présence d'eau et d'une base M(OH)x dans laquelle M est un métal alcalin ou alcalino-terreux, et x est la valence.

2. Procédé selon la revendication 1, où l'étape a) est réalisée en présence (i) d'eau et/ou d'un solvant ionique et/ou d'un solvant eutectique, et (ii) d'un catalyseur acide.

3. Procédé selon la revendication 2, où le catalyseur acide est choisi dans le groupe constitué de : l'acide chlorhydrique, l'acide sulfurique, un acide alkyl sulfurique, un acide sulfonique, un acide alkylsulfonique ou un sulfosuccinate d'alkyl, les acides perhalohydriques, des métaux, leurs oxydes ou leurs sels comme leurs halogénures.

4. Procédé selon la revendication 3, où le catalyseur acide est l'acide méthanesulfonique.

5. Procédé selon l'une quelconque des revendications 1 à 4, où l'alcool ROH est le n-butanol.

6. Procédé selon l'une quelconque des revendications 1 à 5, où l'alcool R'OH est choisi dans le groupe constitué du dodécanol et de l'alcool oléïque.

7. Procédé selon l'une quelconque des revendications 1 à 6, où l'étape d'estérification et glycosylation b) est conduite à la pression atmosphérique et à la température d'ébullition de l'eau ou de l'azéotrope formé avec l'alcool ROH.

8. Procédé selon l'une quelconque des revendications 1 à 7, où l'étape de trans-estérification et trans-glycosylation c) est réalisée à 70°C sous pression réduite afin de recycler l'alcool ROH.

9. Procédé de préparation d'une composition comprenant (i) des sels d'acides alkyl guloside uroniques ou (ii) un mélange de sels d'acides alkyl guloside uroniques et de sels d'acides alkyl mannoside uroniques, selon un procédé tel que défini dans l'une quelconque des revendications 1 à 8, ledit procédé comprenant en outre une étape e) de saponification de l'ester issu de l'étape c).

10. Procédé selon la revendication 9, où l'étape de saponification e) est réalisée en présence (i) d'une base M(OH)x dans laquelle M est un métal alcalin ou alcalino-terreux, et x est la valence, ou (ii) d'une base 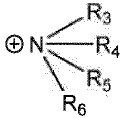 où chacun des R₃ à R₆ est indépendamment un atome d'hydrogène, un alkyle ayant de 1 à 6 atomes de carbone ou un hydroxyalkyle ayant de 1 à 6 atomes de carbone.

11. Procédé selon la revendication 10, où la base est choisie dans le groupe constitué de : l'hydroxyde de sodium, l'hydroxyde de potassium, l'ammoniaque ou un hydroxyde d'alkyl(hydroxyalkyl)ammonium.

12. Procédé selon l'une quelconque des revendications 10 à 11, où l'étape de saponification e) est réalisée à une température de 0 à 100°C.

13. Procédé de préparation d'une composition comprenant (i) des acides alkyl guloside uroniques ou (ii) un mélange d'acides alkyl guloside uroniques et d'acides alkyl mannoside uroniques, selon un procédé tel que défini dans l'une quelconque des revendications 9 à 12, ledit procédé comprenant en outre une étape d'acidification f) des sels issus de l'étape e).

14. Procédé selon la revendication 13, où l'étape d'acidification f) est réalisée en présence d'un acide choisi dans le groupe constitué de :
l'acide chlorhydrique, l'acide sulfurique, un acide sulfonique ou une résine sulfonique sous sa forme H⁺.

15. Composition obtenue par un procédé selon l'une quelconque des revendications 1 à 14.

16. Utilisation d'une composition selon la revendication 15 comme agent tensioactif.

17. Utilisation selon la revendication 16, où l'agent tensioactif est choisi dans le groupe constitué des agents solubilisants, hydrotropes, mouillants, moussants, émulsionnants, émulsifiants et/ou détergents.

18. Tensioactif comprenant une composition selon la revendication 15.

## Patentansprüche

1. Verfahren zur Herstellung einer Zusammensetzung, die Folgendes umfasst:
(i) Alkylgulosid-Alkyluronate nach den Formeln (Ia), (Ib) und (Ic): 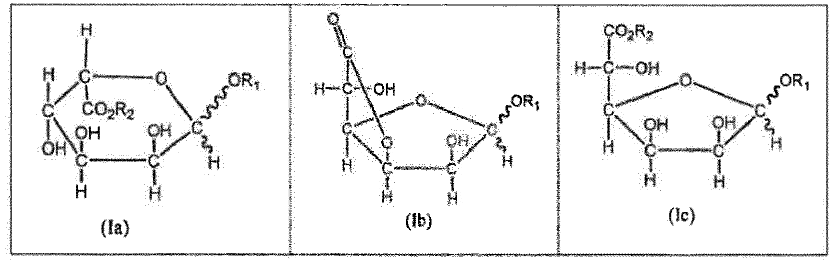 oder
(ii) eine Mischung aus Alkylgulosid-Alkyluronaten nach den Formeln (Ia, Ib und Ic) und Alkylmannosit-Alkyluronaten nach den Formeln (IIa), (IIb) und (IIc): 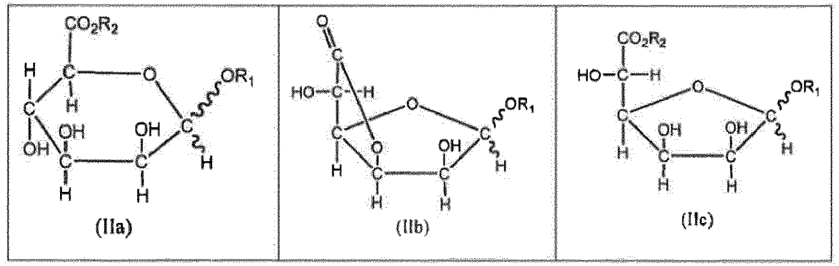 wobei
- R₁ für eine Alkylkette mit 2 bis 22 Kohlenstoffatomen steht, die geradkettig oder verzweigt, gesättigt oder ungesättigt ist;
- R₂ für ein Wasserstoffatom, R₁ für ein Alkalimetallatom, ein Erdalkalimetallatom oder eine quartäre Ammoniumgruppe der Formel (III) steht: 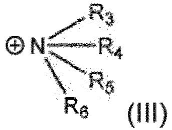
- wobei jedes von R₃ bis R₆ auf unabhängige Weise für ein Wasserstoffatom, ein Alkyl mit 1 bis 6 Kohlenstoffatomen oder ein Hydroxyalkyl mit 1 bis 6 Kohlenstoffatomen steht
und **dadurch gekennzeichnet, dass** das Verfahren Folgendes umfasst:
a) einen Schritt der Hydrolyse von Poly(oligo)guluronaten, Oligoalginaten und/oder Alginaten;
b) einen Schritt der Veresterung und Glykosylierung des Hydrolysats, welches aus dem Schritt a) stammt, mit einem Alkohol der Formel ROH, der geradkettig oder verzweigt, gesättigt oder ungesättigt ist, wobei er 1 bis 4 Kohlenstoffatome aufweist;
c) einen Schritt der Umesterung und Glykosylgruppenübertragung in dem Reaktionsmilieu, welches aus dem Schritt b) stammt, durch einen Alkohol R'OH, der geradkettig oder verzweigt, gesättigt oder ungesättigt ist, wobei er 2 bis 22 Kohlenstoffatom aufweist;
d) möglicherweise einen Schritt der Neutralisierung des Reaktionsmilieus, welches aus dem Schritt c) stammt, in Gegenwart von Wasser und einer Base M(OH)x, wobei M für ein Alkali- oder Erdalkalimetall steht und x die Wertigkeit angibt.

2. Verfahren nach Anspruch 1, wobei der Schritt a) in Gegenwart (i) von Wasser und/oder eines ionischen Lösungsmittels und/oder eines eutektischen Lösungsmittels sowie (ii) eines sauren Katalysators durchgeführt wird.

3. Verfahren nach Anspruch 2, wobei der saure Katalysator aus der Gruppe ausgewählt ist, die aus Folgendem besteht: Salzsäure, Schwefelsäure, einer Alkylschwefelsäure, einer Sulfonsäure, einer Alkylsulfonsäure oder einem Alkylsulfosuccinat, den Perhalogenwasserstoffsäuren, Metallen, deren Oxiden oder deren Salzen wie etwa deren Halogeniden.

4. Verfahren nach Anspruch 3, wobei es sich bei dem sauren Katalysator um Methansulfonsäure handelt.

5. Verfahren nach einem beliebigen der Ansprüche 1 bis 4, wobei es sich bei dem Alkohol ROH um n-Butanol handelt.

6. Verfahren nach einem beliebigen der Ansprüche 1 bis 5, wobei der Alkohol R'OH aus der Gruppe ausgewählt ist, die aus Dodecanol und Oleylalkohol besteht.

7. Verfahren nach einem beliebigen der Ansprüche 1 bis 6, wobei der Schritt b) der Veresterung und Glykosylierung bei Atmosphärendruck und bei der Siedetemperatur von Wasser oder des Azeotrops, welches sich mit dem Alkohol ROH bildet, durchgeführt wird.

8. Verfahren nach einem beliebigen der Ansprüche 1 bis 7, wobei der Schritt c) der Umesterung und der Glykosylgruppenübertragung bei 70 °C unter vermindertem Druck durchgeführt wird, um den Alkohol ROH zurückzuführen.

9. Verfahren zur Herstellung einer Zusammensetzung, die (i) Salze von Alkylgulosid-Uronsäuren oder (ii) eine Mischung aus Salzen von Alkylgulosid-Uronsäuren und Salzen von Alkyl-Mannosid-Uronsäuren umfasst, nach einem Verfahren gemäß der Begriffsbestimmung in einem beliebigen der Ansprüche 1 bis 8, wobei das Verfahren darüber hinaus einen Schritt e) der Verseifung des Esters umfasst, welcher aus dem Schritt c) stammt.

10. Verfahren nach Anspruch 9, wobei der Schritt e) der Verseifung in Gegenwart (i) einer Base M(OH)x, wobei M ein Alkali- oder Erdalkalimetall ist und x die Wertigkeit angibt, oder (ii) einer Base 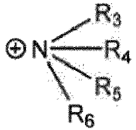 durchgeführt wird, wobei jedes von R₃ bis R₆ auf unabhängige Weise für ein Wasserstoffatom, ein Alkyl mit 1 bis 6 Kohlenstoffatomen oder ein Hydroxyalkyl mit 1 bis 6 Kohlenstoffatomen steht.

11. Verfahren nach Anspruch 10, wobei die Base aus der Gruppe ausgewählt ist, die aus Folgendem besteht: Natriumhydroxid, Kaliumhydroxid, Ammoniumhydroxid oder einem Alkyl(hydroxyalkyl)ammoniumhydroxid.

12. Verfahren nach einem beliebigen der Ansprüche 10 bis 11, wobei der Schritt e) der Verseifung bei einer Temperatur von 0 bis 100 °C durchgeführt wird.

13. Verfahren zur Herstellung einer Zusammensetzung, die (i) Alkylgulosid-Uronsäuren oder (ii) eine Mischung aus Alkylgulosid-Uronsäuren und Alkylmannosid-Uronsäuren umfasst, nach einem Verfahren gemäß der Begriffsbestimmung in einem beliebigen der Ansprüche 9 bis 12, wobei das Verfahren darüber hinaus einen Schritt f) der Ansäuerung der Salze umfasst, welche aus dem Schritt e) stammen.

14. Verfahren nach Anspruch 13, wobei der Schritt f) der Ansäuerung in Gegenwart einer Säure durchgeführt wird, die aus der Gruppe ausgewählt ist, welche aus den folgenden besteht: Salzsäure, Schwefelsäure, einer Sulfonsäure oder einem Sulfonsäureharz in seiner H⁺-Form.

15. Zusammensetzung, die mittels eines Verfahrens nach einem beliebigen der Ansprüche 1 bis 14 erhalten wurde.

16. Verwendung einer Zusammensetzung nach Anspruch 15 als Tensid.

17. Verwendung nach Anspruch 16, wobei das Tensid aus der Gruppe ausgewählt ist, die aus Lösungsvermittlern, hydrotropen Stoffen, Netzmitteln, Schaumbildnern, Emulsionsbildner, Emulgatoren und/oder Detergenzien besteht.

18. Tensid, das eine Zusammensetzung nach Anspruch 15 umfasst.

## Claims

1. Process for preparing a composition comprising:
(i) alkyl(alkyl guloside)uronates of formulae (Ia), (Ib) and (Ic) : 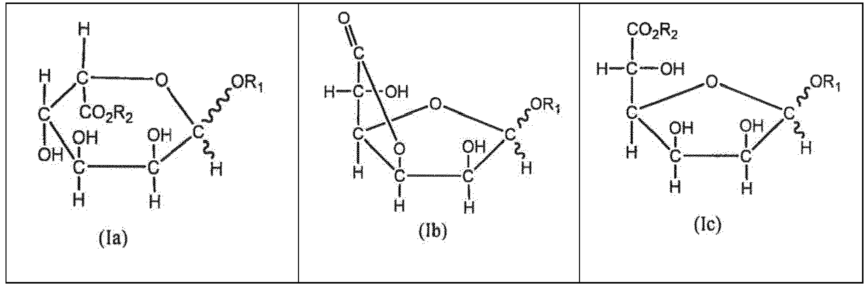 or
(ii) a mixture of alkyl(alkyl guloside)uronates of formulae (Ia), (Ib) and (Ic) and of alkyl(alkyl mannoside)uronates of formulae (IIa), (IIb) and (IIc) : 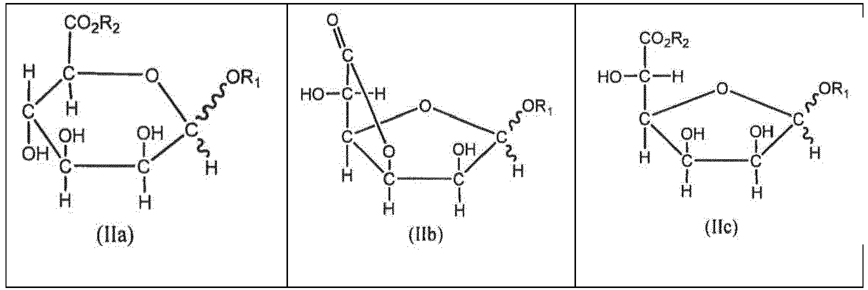 wherein
- R₁ is a linear or branched, saturated or unsaturated alkyl chain having from 2 to 22 carbon atoms;
- R₂ is a hydrogen atom, R₁, an alkali metal atom, an alkaline-earth metal atom, or a quaternary ammonium group of formula (III): 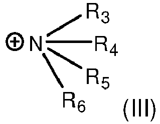
- wherein each of R₃ to R₆ is independently a hydrogen atom, an alkyl having from 1 to 6 carbon atoms or a hydroxyalkyl having from 1 to 6 carbon atoms,
and **characterized in that** said process comprises:
a) a step of hydrolysis of poly(oligo)guluronates, of oligoalginates, of alginates;
b) a step of esterification and glycosylation of the hydrolysate resulting from step a) with a linear or branched, saturated or unsaturated alcohol of formula ROH, having from 1 to 4 carbon atoms;
c) a step of trans-esterification and trans-glycosylation of the reaction medium resulting from step b) with a linear or branched, saturated or unsaturated alcohol of formula R'OH having from 2 to 22 carbon atoms;
d) optionally a step of neutralization of the reaction medium resulting from step c) in the presence of water and of a base M(OH)ₓ in which M is an alkali metal or alkaline-earth metal, and x is the valency.

2. Process as claimed in claim 1, wherein step a) is carried out in the presence (i) of water and/or of an ionic solvent and/or of a eutectic solvent, and (ii) of an acid catalyst.

3. Process as claimed in claim 2, wherein the acid catalyst is chosen from the group consisting of: hydrochloric acid, sulfuric acid, an alkyl sulfuric acid, a sulfonic acid, an alkylsulfonic acid or an alkyl sulfosuccinate, perhalohydric acids, metals, oxides thereof or salts thereof such as halides thereof.

4. Process as claimed in claim 3, wherein the acid catalyst is methanesulfonic acid.

5. Process as claimed in any one of claims 1 to 4, wherein the alcohol ROH is *n-*butanol.

6. Process as claimed in any one of claims 1 to 5, wherein the alcohol R'OH is chosen from the group consisting of dodecanol and oleyl alcohol.

7. Process as claimed in any one of claims 1 to 6, wherein the esterification and glycosylation step b) is carried out at atmospheric pressure and at the boiling point of water or of the azeotrope formed with the alcohol ROH.

8. Process as claimed in any one of claims 1 to 7, wherein the trans-esterification and trans-glycosylation step c) is carried out at 70°C under reduced pressure in order to recycle the alcohol ROH.

9. Process for preparing a composition comprising (i) alkyl guloside uronic acid salts or (ii) a mixture of alkyl guloside uronic acid salts and of alkyl mannoside uronic acid salts, according to a process as defined in any one of claims 1 to 8, said process also comprising a step e) of saponification of the ester resulting from step c).

10. Process as claimed in claim 9, wherein the saponification step e) is carried out in the presence (i) of a base M(OH)ₓ in which M is an alkali or alkaline-earth metal and x is the valency, or (ii) of a base 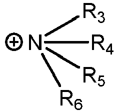 wherein each of R₃ to R₆ is independently a hydrogen atom, an alkyl having from 1 to 6 carbon atoms or a hydroxyalkyl having from 1 to 6 carbon atoms.

11. Process as claimed in claim 10, wherein the base is chosen from the group consisting of: sodium hydroxide, potassium hydroxide, aqueous ammonia or an alkyl(hydroxyalkyl)ammonium hydroxide.

12. Process as claimed in either one of claims 10 and 11, wherein the saponification step e) is carried out at a temperature of from 0 to 100°C.

13. Process for preparing a composition comprising (i) alkyl guloside uronic acids or (ii) a mixture of alkyl guloside uronic acids and alkyl mannoside uronic acids, according to a process as defined in any one of claims 9 to 12, said process also comprising a step of acidification f) of the salts resulting from step e).

14. Process as claimed in claim 13, wherein the acidification step f) is carried out in the presence of an acid chosen from the group consisting of: hydrochloric acid, sulfuric acid, a sulfonic acid or a sulfonic resin in its H⁺ form.

15. Composition obtained by means of a process as claimed in any one of claims 1 to 14.

16. Use of a composition as claimed in claim 15 as a surfactant.

17. Use as claimed in claim 16, wherein the surfactant is chosen from the group consisting of dissolving agents, hydrotropic agents, wetting agents, foaming agents, emulsifiers and/or detergents.

18. Surfactant comprising a composition as claimed in claim 15.
